(19) Europäisches Patentamt
European Patent Office
Office européen des brevets

(11) Veröffentlichungsnummer: **0 417 498 A2**

# (12) EUROPÄISCHE PATENTANMELDUNG

(21) Anmeldenummer: **90115645.5**

(51) Int. Cl.[5]: **C08G 59/50**

(22) Anmeldetag: **16.08.90**

(30) Priorität: **14.09.89 DE 3930718**

(43) Veröffentlichungstag der Anmeldung:
**20.03.91 Patentblatt 91/12**

(84) Benannte Vertragsstaaten:
**AT BE CH DE ES FR GB IT LI NL SE**

(71) Anmelder: **SCHERING AKTIENGESELLSCHAFT**
**Berlin und Bergkamen**
**Waldstrasse 14**
**W-4709 Bergkamen(DE)**

(72) Erfinder: **Burba, Christian, Dr. Dipl.-Chem.**
**Gerhart-Hauptmann-Strasse 9**
**W-4715 Herbern(DE)**
Erfinder: **Mrotzek, Werner, Dr. Dipl.-Ing.**
**Dresdener Strasse 24**
**W-4600 Dortmund 1(DE)**

## (54) Imidazolylderivate, ihre Verwendung als Härtungsmittel in Epoxidharzzusammensetzungen, sie enthaltende härtbare Epoxidharzzusammensetzungen und Epoxidharzformkörper.

(57) Die Erfindung betrifft neue Imidazolylderivate, ihre Verwendung als Härtungsmittel in Epoxidharzzusammensetzungen, sie enthaltende härtbare Epoxidharzzusammensetzungen, bestehend aus einem Epoxidharz und aus den Verbindungen der allgemeinen Formel I

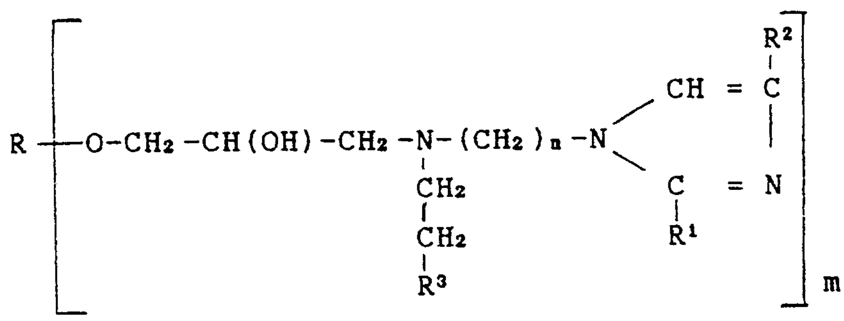

(I)

worin R der Rest der zur Herstellung der Glycidylether verwendeten Alkoholkomponente is, $R^1$ und $R^2$ unabhängig voneinander Wasserstoff, $-CH_3$, $C_2H_5$ bedeuten und $_n$ = 2 oder 3 ist, $R^3$ = COOH, -CN, -CONH-$NH_2$, - $COOCH_2$-$CH_2$-OH, -$COOR^4$ mit $R^4$ = aliphatischer Kohlenwasserstoffrest mit 1 - 4 C-Atomen und m gleich der Wertigkeit von R ist.

EP 0 417 498 A2

# IMIDAZOLYLDERIVATE, IHRE VERWENDUNG ALS HÄRTUNGSMITTEL IN EPOXIDHARZZUSAMMENSETZUNGEN, SIE ENTHALTENDE HÄRTBARE EPOXIDHARZZUSAMMENSETZUNGEN UND EPOXIDHARZFORMKÖRPER

Gegenstand der Erfindung sind neue Imidazolylderivate, ihre Verwendung als Härtungsmittel in Epoxidharzzusammensetzungen, sie enthaltende härtbare Epoxidharzzusammensetzungen, bestehend aus einem Epoxidharz und aus den Verbindungen der allgemeinen Formel I und gegebenenfalls üblichen Härtungs- und Lösungsmitteln zur Herstellung von Formkörpern.

Für die Herstellung von Verbundwerkstoffen werden heute im Prinzip zwei Verfahren angewandt.

Bei dem Naß-in-Naß-Verfahren werden die Verstärkungsmaterialien mit der härtbaren Mischung imprägniert und in der Hitze in einer Stufe bis zum duromeren Endzustand ausgehärtet.

Beim Zweistufenverfahren werden aus den Verstärkungsmaterialien und der härtbaren Mischung zunächst sogenannte Prepregs hergestellt, die dann in einem zeitlich getrennten 2. Verfahrensschritt zu Fertigteilen weiterverarbeitet werden. Dabei ist verfahrenstechnisch noch einmal zwischen der Verwendung lösungsmittelhaltiger und lösungsmittelfreier Arbeitsweise zu unterscheiden.

Die Herstellung der Prepregs erfolgt normalerweise in einem kontinuierlichen Prozeß, in dem die Verstärkungsmaterialien entweder durch ein Imprägnierbad des einzusetzenden Harz-Härter-Gemisches geleitet werden oder das Imprägniermittel wird erst unmittelbar vor seinem Auftrag auf das Trägermaterial vermischt und mittels einer besonderen Vorrichtung aufgerakelt. Die Steuerung der auf eine bestimmte Trägermaterialbahn aufzubringenden Imprägniermittelmenge erfolgt dabei außer über die Viskosität des Imprägniermittels über nachgeschaltete Abquetschwalzen.

Bei lösungsmittelhaltigen Systemen wird nach dem Imprägnierprozeß durch Wärmezufuhr das in der Imprägnierlösung enthaltende Lösungsmittel verdampft und gleichzeitig das Harzsystem vom A- in den B-Zustand überführt. Aus den mit flüssigem bis stark klebrigem Imprägniermittel getränkten Verstärkungsmaterialien wird je nach Verfahrensbedingungen und verwendetem Harzsystem ein schwach klebriger bis fast trockener Prepreg. Bei diesem Verfahrensschritt ist es wichtig, daß einerseits das Lösungsmittel der Imprägniermischung vollständig entzogen wird, andererseits aber der für die Prepreg-Härtung im 2. Verfahrensschritt notwendige latente Härter noch nicht anspringt, um ein ungewolltes Ausreagieren der imprägnierten Verstärkungsmaterialien zu verhindern.

Bei lösungsmittelfreien Systemen erfolgt abhängig von der chemischen Zusammensetzung des Harzsystems nach der Imprägnierung entweder ebenfalls eine kurze Wärmebehandlung des Materials, oder die Verstärkungsstoffe werden unmittelbar nach der Imprägnierung unter Verzicht auf eine gesonderte Wärmebehandlung beidseitig mit Trennfolien kaschiert und einer systemadäquaten Zwischenlagerung zugeführt. Im Verlaufe dieser Zwischenlagerung tritt entweder ein allmählicher Übergang des Harzsystems in den B-Zustand ein oder das Imprägniermittel wird unter weitgehendem Verzicht auf chemische Veränderungen allein durch physikalische Effekte auf den Trägermaterialien fixiert.

Die so erhaltenen Prepregs lassen sich als Rollen zwischenlagern und transportieren, ehe sie dem jeweiligen Anwendungsfall entsprechend zugeschnitten und in Bauteildicke übereinander geschichtet werden. Durch gleichzeitige Druck-und Temperatureinwirkung wird der Prepregstapel zu einem hochfesten Formteil ausgehärtet, wobei die noch niedermolekularen, fließfähigen Harze in den hochmolekularen C-Zustand des Duromers übergehen.

Während beim Einstufenverfahren lange offene Zeiten und kurze Aushärtungszeiten bei niedrigen Härtungstemperaturen gefordert werden, kommt beim Zweistufenverfahren als weiteres Kriterium noch eine möglichst lange Lagerstabilität der Prepregs hinzu. Dabei werden Lagerungstemperaturen unterhalb Raumtemperatur von der Praxis immer weniger akzeptiert.

Von Bedeutung ist weiterhin, daß je nach Herstellungsverfahren der Prepregs die Materialviskosität der gebrauchsfertigen härtbaren Mischung über einen möglichst langen Zeitraum weitgehend unverändert bleibt. Speziell bei Verwendung eines großvolumigen Tränkbades ist dies für die Erzielung eines konstanten Harzauftrages und eines gleichbleibenden B-Zustandes erforderlich, da zum einen die Bedingungen der Produktion nicht laufend den sich ändernden Verhältnissen in der härtbaren Mischung angepaßt werden können und zum anderen dadurch auch die physikalischen Eigenschaften des ausgehärteten Endproduktes negativ beeinflußt werden.

Angestrebt wird in der Praxis eine härtbare Mischung, welche im Imprägnierbad auch über längere Zeit viskositätsmäßig konstant bleibt und dann als Prepreg bei Raumtemperatur ohne chemische Veränderungen lange gelagert werden kann.

Unabhängig von der Prepregherstellung soll ihre Aushärtung bei möglichst tiefen Temperaturen innerhalb kurzer Zeit erfolgen, das Temperaturmaximum der exothermen Reaktion auch bei größeren

Schichtdicken auf einem niedrigen Niveau verbleiben und das physikalische Eigenschaftsniveau der Endprodukte den Erfordernissen der Praxis angepaßt sein.

Diese Forderungen hinsichtlich des Härtungsverhaltens und Eigenschaftsniveaus gelten in gleicher Weise auch für die im Naß-in-Naß-Verfahren zu verarbeitenden Epoxidharzsysteme.

Das in härtbaren Mischungen auf Basis von Epoxidharzen seit langem als latenter Härter verwendete Dicyandiamid wird zur Erzielung der angestrebten Eigenschaften in der Regel mit Co-Härtungsmitteln und/oder Beschleunigern kombiniert. Aus der Literatur sind auf diesem Gebiet daher eine Vielzahl von Vorschlägen bekannt geworden.

Bei Verwendung von Dicyandiamidlösungen können zwar homogene Substrate hergestellt werden, jedoch bringt die Verwendung von Lösungsmitteln andere Probleme mit sich.

Dicyandiamid ist nur in wenigen Lösungsmitteln wie insbesondere Dimethylformamid oder Methylglykol in ausreichenden Mengen löslich. Diese Lösungsmittel sind jedoch toxikolo gisch bedenklich und bringen sowohl bei der Herstellung der Prepregs, das heißt bei Imprägnierung der Verstärkungsmaterialien und Überführung in den B-Zustand, als auch bei der Entsorgung Probleme mit sich.

Aufgrund der Schwerlöslichkeit des Dicyandiamids müssen größere Mengen an Lösungsmittel verwendet werden, welche die Tränkviskosität so beeinflussen, daß der Bindemittelgehalt auf den Verstärkungsmaterialien nicht beliebig gewählt werden kann.

Da diese Lösungsmittel bei der Härtung nicht vollständig entfernt werden können, besteht bei einer thermischen Belastung der Bauteile weiterhin die Gefahr, daß der Werkstoff vorzeitig versagt und/oder die Lösungsmittel vor Ort unkontrolliert an die Umgebungsluft abgegeben werden.

Bei Einsatz von festem kristallinen Dicyandiamid in flüssigen Epoxidharzen ohne Mitverwendung von Lösungsmitteln wird das Dicyandiamid entweder direkt in der erforderlichen Menge im Epoxidharz dispergiert oder es wird zuvor eine hochgefüllte Dicyandiamid/Epoxidharz-Paste hergestellt, welche zu einem späteren Zeitpunkt mit der Hauptmenge Epoxidharz auf die gewünschten Harz/Härter-Konzentrationen eingestellt wird.

In jedem Falle ist hier die Herstellung der Dispersionen nicht einfach, und außerdem neigen diese bei längeren Standzeiten insbesondere unter Imprägnierbedingungen zur Separation.

Bei Einsatz von festem kristallinen Dicyandiamid in bei Raumtemperatur festen Epoxidharzen ohne Mitverwendung von Lösungsmitteln wird in erster Stufe wiederum eine Paste aus Dicyandiamid und flüssigen Epoxidharzen hergestellt, welche dann bei erhöhter Temperatur in die Festharzschmelze eingearbeitet wird.

Neben den bereits oben angeführten Problemen werden bei dieser Vorgehensweise in das Festharz unverwünschte Anteile von flüssigen Epoxidharzen eingebracht.

Weiterhin werden bei Einsatz von festem kristallinen Dicyandiamid in den gehärteten Substraten Inhomogenitäten festgestellt, welche auf nicht gelösten und nicht umgesetzten Partikeln beruhen.

Aufgabe der vorliegenden Erfindung war es, unter Vermeidung der Nachteile des Standes der Technik härtbare Mischungen auf Basis von Epoxidverbindungen und flüssigen latenten und in den Epoxidharzen löslichen bzw. homogen verteilbaren Härtungsmitteln zu finden, welche bei relativ niedrigen Temperaturen innerhalb kurzer Zeit ohne hohe exotherme Temperaturspitzen zum duromeren Endzustand aushärten, eine den Anforderungen der Praxis entsprechende thermische Beständigkeit besitzen und welche in Prepregs bei Raumtemperatur über eine ausreichende Lagerstabilität verfügen.

Diese Aufgabe wird gelöst durch Verwendung eines neuen Härtungsmittels, gegebenenfalls unter Mitverwendung üblicher latenter Härtungsmittel.

Gegenstand der Erfindung sind daher Verbindungen der allgemeinen Formel (I)

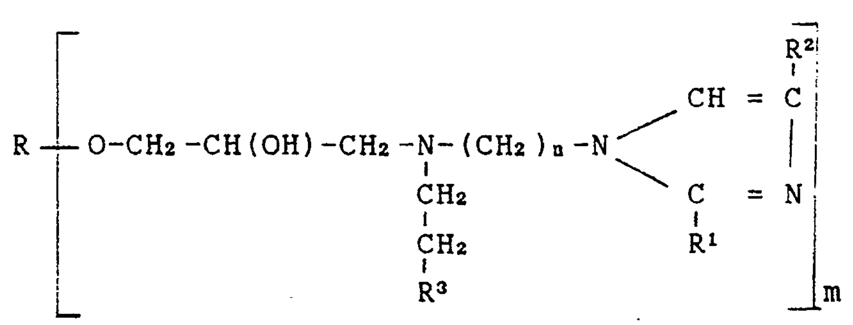

(I)

worin R der Rest der zur Herstellung der Glycidylether verwendeten Alkoholkomponente, insbesondere der

Polypropylenglykolrest mit einem Molgewicht von 500 - 1000 ist, $R^1$ und $R^2$ unabhängig voneinander Wasserstoff, $-CH_3$, $-C_2H_5$ bedeuten und $_n$ = 2, insbesondere 3 ist, $R^3$ = COOH, -CN, $-CONH-NH_2$, $-COOCH_2-CH_2-OH$, $COOR^4$ mit $R^4$ = aliphatischer Kohlenwasserstoffrest mit 1 - 4 C-Atomen, insbesondere $-CH_3$, $-C_2H_5$ und m gleich der Wertigkeit von R, vorzugsweise 1 bis 3 ist.

Ein weiterer Gegenstand der Erfindung ist die Verwendung von Verbindungen der allgemeinen Formel (I)

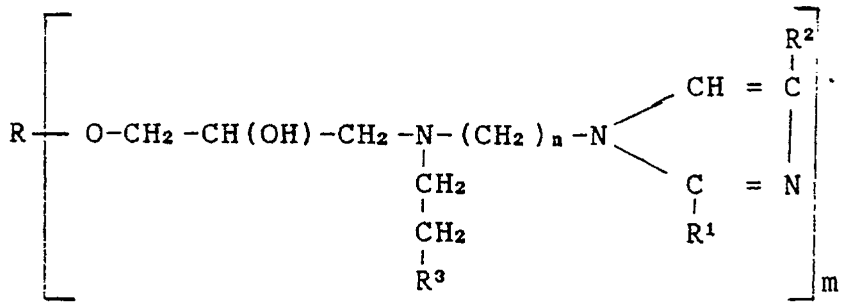

(I)

worin R der Rest der zur Herstellung der Glycidylether verwendeten Alkoholkomponente, insbesondere der Polypropylenglykolrest mit einem Molgewicht von 500 - 1000 ist, $R^1$ und $R^2$ unabhängig voneinander Wasserstoff, $-CH_3$, $-C_2H_5$ bedeuten und $_n$ = 2, insbesondere 3 ist, $R^3$ = COOH, -CN, $-CONH-NH_2$, $-COOCH_2-CH_2-OH$, $COOR^4$ mit $R^4$ = aliphatischer Kohlenwasserstoffrest mit 1 - 4 C-Atomen, insbesondere $-CH_3$, $-C_2H_5$ und m gleich der Wertigkeit von R, vorzugsweise 1 bis 3 ist, gegebenenfalls unter Mitverwendung üblicher stickstoffhaltiger heterocyclischer Aminverbindungen als Härtungsmittel für Epoxidharze.

Ein weiterer Gegenstand der Erfindung sind härtbare Epoxidharz-Zusammensetzungen, enthaltend

a) ein Epoxidharz mit durchschnittlich mehr als einer Epoxidgruppe pro Molekül und

b) Verbindungen der allgemeinen Formel (I)

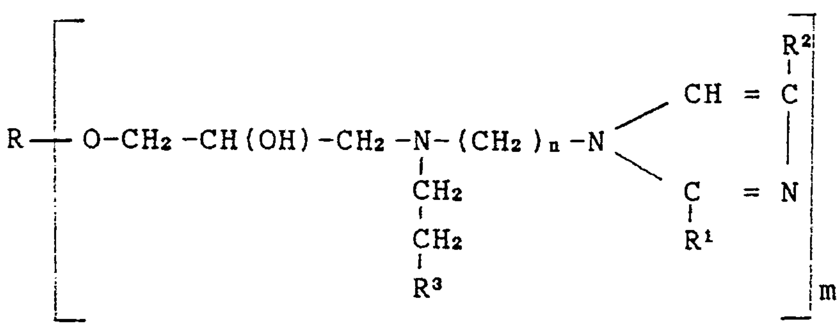

(I)

worin R der Rest der zur Herstellung der Glycidylether verwendeten Alkoholkomponente, insbesondere der Polypropylenglykolrest mit einem Molgewicht von 500 -1000 ist, $R^1$ und $R^2$ unabhängig voneinander Wasserstoff, $-CH_3$, $-C_2H_5$ bedeuten und $_n$ = 2, insbesondere 3 ist, $R^3$ = COOH, -CN, $-CONH-NH_2$, $-COOCH_2-CH_2-OH$, $COOR^4$ mit $R^4$ = aliphatischer Kohlenwasserstoffrest mit 1 - 4 C-Atomen, insbesondere $-CH_3$, $-C_2H_5$ und m gleich der Wertigkeit von R, vorzugsweise 1 bis 3 ist und gegebenenfalls

c) übliche Lösungsmittel, Füllstoffe, Verstärkungs- oder Einlagematerialien, Pigmente, Hilfsstoffe und gegebenenfalls

d) übliche stickstoffhaltige heterocyclische Aminverbindungen.

Ein weiterer Gegenstand der Erfindung sind härtbare Epoxidharz-Zusammensetzungen, worin die Verstärkungs- oder Einlagematerialien bei Raumtemperatur mit dem Bindemittel, bestehend aus

a) einem Epoxidharz mit durchschnittlich mehr als einer Epoxidgruppe pro Molekül und

b) Verbindungen der allgemeinen Formel (I)

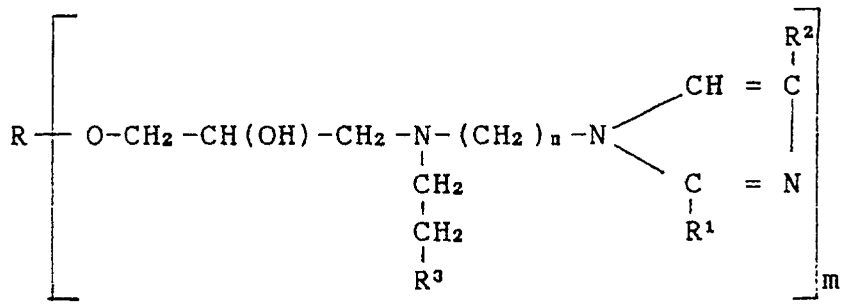

(I)

worin R der Rest der zur Herstellung der Glycidylether verwendeten Alkoholkomponente, insbesondere der Polypropylenglykolrest mit einem Molgewicht von 500 - 1000 ist, $R^1$ und $R^2$ unabhängig voneinander Wasserstoff, $-CH_3$, $-C_2H_5$ bedeuten und $_n$ = 2, insbesondere 3 ist, $R^3$ = COOH, -CN, $-CONH-NH_2$, $-COOCH_2-CH_2-OH$, $COOR^4$ mit $R^4$ = aliphatischer Kohlenwasserstoffrest mit 1 - 4 C-Atomen, insbesondere $-CH_3$, $-C_2H_5$ und m gleich der Wertigkeit von R, vorzugsweise 1 bis 3 ist und gegebenenfalls

c) Lösungsmitteln, Füllstoffen, Verstärkungs- oder Einlagematerialien, Pigmenten, Hilfsstoffen und gegebenenfalls

d) üblichen stickstoffhaltigen heterocyclischen Aminverbindungen getränkt und gegebenenfalls bei erhöhter Temperatur in den halbfesten aber noch schmelzbaren Zustand (B-Zustand) überführt werden.

Ein weiterer Gegenstand der Erfindung sind Epoxidharzformkörper, dadurch gekennzeichnet, daß in erster Stufe die Verstärkungs- oder Einlagematerialien bei Raumtemperatur mit dem Bindemittel, bestehend aus

a) einem Epoxidharz mit durchschnittlich mehr als einer Epoxidgruppe pro Molekül

b) Verbindungen der allgemeinen Formel (I)

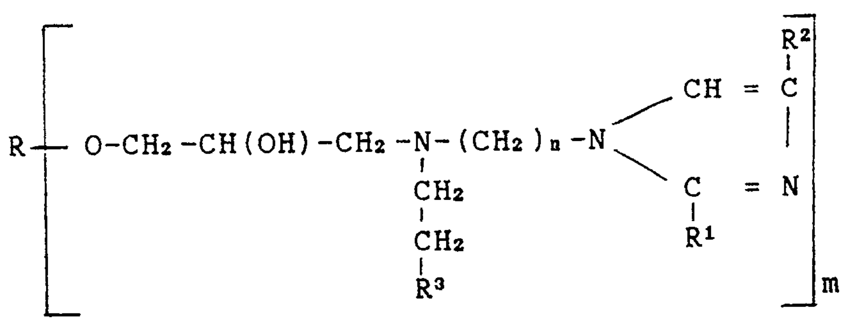

(I)

worin R der Rest der zur Herstellung der Glycidylether verwendeten Alkoholkomponente, insbesondere der Polypropylenglykolrest mit einem Molgewicht von 500 - 1000 ist, $R^1$ und $R^2$ unabhängig voneinander Wasserstoff, $-CH_3$, $-C_2H_5$ bedeuten und $_n$ = 2, insbesondere 3 ist, $R^3$ = COOH, -CN, $-CONH-NH_2$, $-COOCH_2-CH_2-OH$, $COOR^4$ mit $R^4$ = aliphatischer Kohlenwasserstoffrest mit 1 - 4 C-Atomen, insbesondere $-CH_3$, $-C_2H_5$ und m gleich der Wertigkeit von R, vorzugsweise 1 bis 3 ist und gegebenenfalls

c) übliche Lösungsmittel, Füllstoffe, Verstärkungs- oder Einlagematerialien, Pigmente, Hilfsstoffe und gegebenenfalls

d) üblichen stickstoffhaltigen heterocyclischen Aminverbindungen getränkt und gegebenenfalls in den halbfesten, aber noch schmelzbaren Zustand (B-Zustand) überführt werden und in zweiter Stufe die feuchten Laminate oder Prepregs unter Formgebung oder zwischen zu verklebende Substrate gebracht und unter Anwendung von Druck bei erhöhter Temperatur ausgehärtet werden.

Die erfindungsgemäßen Imidazolylverbindungen sind herstellbar durch einfache Additionsreaktionen, wobei man in erster Stufe einen Ethergruppen enthaltenden Glycidylether mit primären Aminogruppen enthaltenden N-Aminoalkylimidazolyl-Verbindungen umsetzt, wobei das Verhältnis von Epoxidgruppen zu primären Aminogruppen ca. 1 : 1 beträgt, und in zweiter Stufe an die entstandenen sekundären Aminogruppen Acrylsäure oder deren Derivate addiert. Die Additionsreaktionen der ersten und zweiten Stufe werden nach den bekannten Verfahren durchgeführt.

Die erfindungsgemäß zur Herstellung der Imidazolylverbindungen mitverwendeten Ethergruppen enthaltenden Glyci dylether sind Umsetzungsprodukte von ein- oder mehrwertigen Alkoholen und Epichlorhydrin zu den Chlorhydrinethern und anschließender Ringbildung mit Alkalihydroxiden. Diese Reaktionen erfolgen

gemäß den zum bekannten Stand der Technik gehörenden Verfahren.

Als Alkohole können hier deren Ether- bzw. Estergruppen enthaltenden höhermolekularen Folgeprodukte eingesetzt werden.

Üblicherweise finden Polyetherpolyole Verwendung, die durch anionische Polymerisation, Copolymerisation und Blockcopolymerisation von Alkylenoxiden, wie Ethylenoxid, Propylenoxid und Butylenoxid, mit mono- bis- oder polyfunktionellen Alkoholen, wie Butandiol-(1,4), 1,1,1-Trimethyloläthan, 1,1,1-Trimethylolpropan, Hexantriol-(1,2,6), Glycerin, Pentaerythrit und Sorbit, oder mit Aminen, wie Methylamin, Äthylendiamin und 1,6-Hexamethylendiamin, als Startkomponenten oder durch kationische Polymerisation und Copolymerisation cyclischer Ether, wie Tetrahydrofuran, Ethylenoxid und Propylenoxid, mit sauren Katalysatoren, wie Bortrifluoridätherat und durch Polykondensation von unter Wasserabspaltung polykondensierbaren Glykolen, wie Hexandiol-(1,6) in Gegenwart saurer Veretherungskatalysatoren, wie p-Toluolsulfonsäure, erhalten werden, sowie, z. B. im Hinblick auf eine Flammschutzwirkung, Oxalkylierungsprodukte der Phosphorsäure und phosphoriger Säuren, z. B. mit Ethylenoxid, Propylenoxid, Butylenoxid und Styroloxid. Als Polythioetherpolyole kommen vorzugsweise die Polykondensationsprodukte des Thioglykols mit sich und mit Diolen und/oder Polyolen, wie z. B. Hexandiol-(1,6), Triethylenglykol, 2,2-Dimethyl-propandiol-(1,3) und 1,1,1-Tri methylolpropan, in gegenwart saurer Veretherungskatalysatoren, wie Phosphorsäure und phosphorige Säure, in Betracht. Als Polyacetale seien vorzugsweise die Polykondensationsprodukte aus Formaldehyd und Diolen und/oder Polyolen, wie Diethylenglykol, Triethylenglykol, Butandiol-(1,4), Hexandiol-(1,6), Thioglykol und 1,1,1-Trimethylolpropan, mit sauren Katalysatoren, wie Phosphorsäure und p-Toluolsulfonsäure, genannt. Als Polyesterpolyole sind vorzugsweise die Kondensationsprodukte mit Di- und/oder Polycarbonsäuren und Di- und/oder Polyolen, die durch Polykondensation z. B. von Adipinsäure, Phthalsäure, Tetrahydrophthalsäure, Hexahydrophthalsäure und Ethylenglykol, Butandiol-(1,4), Diethylenglykol, Triethylenglykol, Hexandiol-(1,6), 2,2-Dimethyl-propandiol-(1,2,6) hergestellt werden, ferner Polycarbonate der genannten Di- und Polyole und Polyesteramide unter zusätzlicher Verwendung von Aminoalkoholen, wie z. B. $\alpha$-Caprolacton geeignet.

Die Ausgangs-Alkohole weisen Molgewichte von ca. 100 -2.000 auf. Erfindungsgemäß bevorzugt werden Polypropylenglykole mit Molgewichten von ca. 500 - 1.000.

Die erfindungsgemäß mitverwendeten Imidazolylverbindungen sind Verbindungen der allgemeinen Formel II

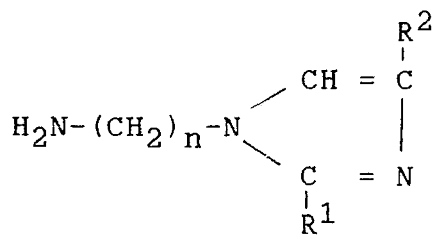

worin $R^1$ und $R^2$ unabhängig voneinander aliphatische oder aromatische Kohlenwasserstoff-Reste, insbesondere aber H-, $CH_3$, $C_2H_5$- bedeuten und n = 2 und insbesondere = 3 ist. Pro Epoxidgruppe der oben angeführten Glycidylverbindungen wird 1 mol der Imidazolylverbindung eingesetzt.

Die erfindungsgemäß mitverwendeten Acrylsäure- bzw. Acrylsäurederivate sind Verbindungen der allgmeinen Formel III

$CH_2 = CH\text{-}R^3$

mit $R^3$ = -COOH, -CN, -CONH-$NH_2$, -$COOC_2H_4OH$, -$COOR_4$ mit $R^4$ = aliphatischer Kohlenwasserstoffrest mit 1 - 4 C-Atomen, insbesondere -$CH_3$ oder -$C_2H_5$.

Pro sekundärer Aminogruppe der in erster Stufe hergestellten Additionsverbindungen wird 1 Mol der Acrylsäureverbindungen eingesetzt.

Die gemäß diesen Verfahrensschritten herstellbaren erfindungsgemäßen Additionsprodukte sind Verbindungen der allgemeinen Formel I

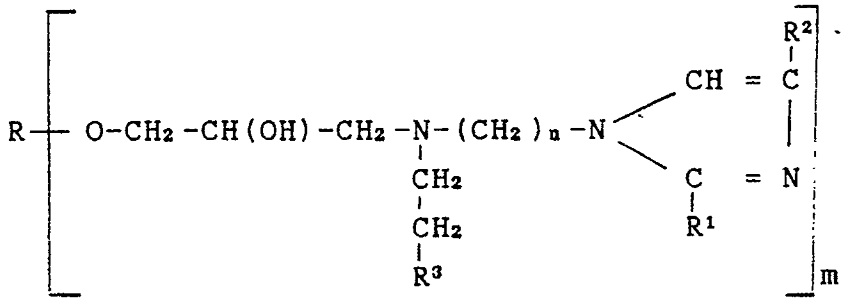

(I)

worin R der Rest der zur Herstellung der Glycidylether verwendeten Alkoholkomponente, insbesondere der Polypropylenglykolrest mit einem Molgewicht von 500 - 1000 ist, $R^1$ und $R^2$ unabhängig voneinander Wasserstoff, $-CH_3$, $-C_2H_5$ bedeuten und $_n$ = 2, insbesondere 3 ist, $R^3$ = COOH, -CN, $-CONH-NH_2$, $-COOCH_2-CH_2-OH$, $COOR^4$ mit $R^4$ = aliphatischer Kohlenwasserstoffrest mit 1 - 4 C-Atomen, insbesondere $-CH_3$, $-C_2H_5$ und m gleich der Wertigkeit von R, vorzugsweise 1 bis 3 ist.

Die erfindungsgemäßen Härtungsmittel können allein oder als Mischung eingesetzt werden, wobei pro 100 g Epoxidharz 5 -35 g, vorzugsweise 7 - 25 g, insbesondere aber 10 - 20 g Härtungsmittel mitverwendet werden.

Es ist ebenfalls möglich, die Verbindungen der allgemeinen Formel I in Form ihrer Salze einzusetzen. Verwendbar sind hier die auf diesem Gebiet bekannten organischen und anorganischen Salzbildner. Bevorzugt werden erfindungsgemäß jedoch die ein- oder mehrwertigen organischen Carbonsäuren, wobei insbesondere die verzweigten Monocarbonsäuren mit bis zu 10 C-Atomen wie 2-Ethylhexansäure eingesetzt werden können.

Die erfindungsgemäß als Bindemittelbestandteil mitverwendeten Epoxidharze sind Glycidylester und -ether mit zwei oder mehr Epoxidgruppen pro Molekül wie vorzugsweise die Glycidylether auf Basis von ein- oder mehrwertigen Phenolen. Erfindungsgemäß bevorzugt werden Glycidylether von 2,2-Bis(4-hydroxyphenyl)-propan (Bisphenol A) mit Epoxidwerten von 0,2 - 0,6, insbesondere die bei Raumtemperatur flüssigen Verbindungen mit Epoxidwerten um 0,45 bis 0,55. Daneben haben sich auch die Glycidylether auf Basis von Bisphenol F und die Novolake als vorteilhaft erwiesen.

Die handelsüblichen halogenierten, insbesondere bromierten Epoxidharze auf Basis der oben aufgeführten Phenole sind ebenfalls verwendbar.

Als erfindungsgemäß gegebenenfalls mitzuverwendende Aminverbindungen können vorzugsweise übliche stickstoffhaltige heterocyclische Aminverbindungen, das heißt N-Alkylimidazole wie N-Methyl-, N-Ethylimidazol und/oder Imidazolinverbindungen der allgemeinen Formel IV

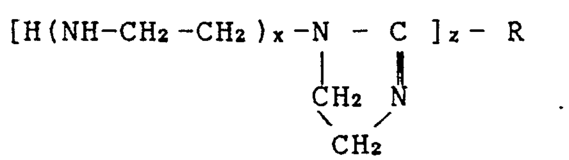

IV

worin R ein gegebenenfalls verzweigter Alkyl- oder Alkylenrest mit < 10 C-Atomen, insbesondere $-CH_3$, $-CHOH-CH_3$, $-(CHR')-_y$, worin R' = H oder $-CH_3$ bedeuten und $_x$ = 1, 2, 3 und $_y$ = 4 - 8 und z gleich der Wertigkeit von R ist, mitverwendet werden, insbesondere solche, in denen $_x$ = 1, $_z$ = 1 und R = $-CH_3$ oder $-CH_2-CH_3$ ist. Andere auf diesem Gebiet übliche Härtungsmittel können, falls gewünscht, ebenfalls mitverwendet werden.

Zur Modifizierung der Eigenschaften des Endprodukts können neben anderen Epoxidharzen auch Modifizierungs- oder Hilfsstoffe mitverwendet werden wie Phenolharze, Melaminharze, Silikonharze, anorganische und organische Füllstoffe wie Quarzmehle, Titandioxid, Ruß, Silikon- oder Butandienkautschuk.

Zur Einstellung der gewünschten Viskosität können unterschiedlich viskose Harze oder Verdünnungsmittel mitverwendet werden, aber auch die üblichen Lösungsmittel wie Di methylformamid, Aceton, Methylethylketon, Methylglykol, Propylenglykolmonomethylether oder deren Mischungen eingesetzt werden.

Zur Herstellung der Prepregs werden organische und anorganische Fasern, Vliese und Gewebe auf Basis von Aramid, Kohlenstoff oder Cellulose, Metallen wie Bor, Stahl usw., Keramik, insbesondere aber Glas, mitverwendet.

Die Herstellung der lösungsmittelhaltigen Prepregs geschieht nach der an sich bekannten Methode, bei der die Trägermaterialien in einem Imprägnierbad mit der reaktiven Harzmischung getränkt und nach dem Abquetschen der überschüssigen Harzmenge kontinuierlich unter Energiezufuhr (meist Wärme), bei gleichzeitigem Entzug des Lösungsmittels, vom A- in den B-Zustand überführt werden. Je nach gewünschter Prepreg-Konsistenz (klebrig bis fest) werden diese anschließend beidseitig mit einer Trennfolie versehen und für die Lagerung und den Transport aufgerollt. Die Weiterverarbeitung erfolgt durch Zuschneiden und Zusammenlegen der einzelnen Prepreg-Lagen zu einem Stapel, aus dem durch formgebende Maßnahmen unter gleichzeitiger Wärmezufuhr ein hochvernetztes Werkstück entsteht.

Die erfindungsgemäßen Härtungsmittel können außerdem mit Erfolg in lösungsmittelfreien Prepregs auf Basis von Epoxidharzen und gegebenenfalls üblichen Härtungsmitteln verwendet werden. Hierbei werden die Trägermaterialien bei gegebebenenfalls erhöhter Temperatur nach an sich bekannten Verfahren mit den Bindemittelsystemen getränkt und systemadäquat gelagert, ehe sie wie die Lösungsmittel enthaltenden Systeme weiterverarbeitet werden.

Weitere Beispiele für lösungsmittelfreie Systeme sind Naßlaminate, Trägermaterialien für den Elektrosektor, insitu hergestellte faserverstärkte Formteile (RTM), heißhärtende Einkomponentenklebstoffe z. B. für die Verklebung von Karosserieteilen in der Automobilindustrie (Bördelnahtkleber) und Epoxidharzgießlinge, Epoxidharz-Beschichtungsmittel oder -wickelkörper.

## Beispiele

### I. Herstellung der erfindungsgemäßen Härter

#### Beispiel I.1

a) 125 g (1 Mol) 1-(3-Aminopropyl)imidazol werden unter Stickstoff vorgelegt, auf ca. 70 °C vorgewärmt und mit 475 g des Diglycidylethers des Polypropylenglykol 620 (Epoxid-Äquivalent 475) langsam versetzt. Anschließend wird nach Abklingen der exothermen Reaktion 2 h/70 °C nachgerührt.

b) Zu dem unter a) hergestellten Addukt werden bei ca. 50 °C 86 g (1 Mol) Acrylsäuremethylester dosiert und ca. 2,5 h/60 °C nachgerührt.

c) Zu dem unter b) beschriebenen Additionsprodukt werden 62,5 g Hydrazinhydrat 80 %ig in Wasser (entspr. 1 Mol Hydrazin) bei 50 - 60 °C zugegeben und 2 h/70 °C nachgerührt. Anschließend wird auf 90 °C aufgeheizt und nach 1 h/90 °C vorsichtig Vakuum gezogen bis ca. 10 mbar, um Wasser und Methanol zu entfernen.

Kenndaten: Aminzahl: 226/224
Viskosität/25 °C: 38,0 Pa.s

#### Beispiel I.2

a) 125 g (1 Mol) 1-(3-Aminopropyl)imidazol werden unter Stickstoff vorgelegt, auf ca. 70 °C vorgewärmt und mit 475 g des Diglycidylethers des Polypropylenglykol 620 (Epoxid-Äquivalent 475) langsam versetzt. Anschließend wird nach Abklingen der exothermen Reaktion 2 h/70 °C nachgerührt.

b) Zu dem unter a) hergestellten Addukt werden bei ca. 60 °C 116 g (1 Mol) 2-Hydroxiethylacrylat dosiert und ca. 2,5 h/60 °C nachgerührt.

Kenndaten: Aminzahl: 144/145
Viskosität/25 °C: ca. 22,0 Pa.s

#### Beispiel I.3

Man verfährt wie unter Beispiel I.1 a,b,c beschrieben, lediglich mit dem Unteschied, daß anstelle von 475 g Diglycidylether des Polypropylenglykol 620 158 g Hexandioldiglycidylether (Epoxid-Äquivalent 158) eingesetzt werden.

Kenndaten: Aminzahl: 265/268
Viskosität/25 °C: ca. 14,5 Pa.s

Beispiel I.4

a) 125 g (1 Mol) 1-(3-Aminopropyl)imidazol werden unter Stickstoff vorgelegt, auf ca. 70 °C vorgewärmt und mit 714 g des Triglycidylethers des trifunktionellen Polypropylenglykols (Epoxid-Äquivalent 714) langsam versetzt. Anschließend wird nach Abklingen der exothermen Reaktion 2 h/70 °C nachgerührt.
b) Zu dem unter a) hergestellten Addukt werden bei ca. 50 °C 86 g (1 Mol) Acrylsäuremethylester dosiert und ca. 2,5 h/60 °C nachgerührt.
Kenndaten: Aminzahl: 114/115
Viskosität/25 °C: ca. 3,5 Pa.s

Beispiel I.5

Zu 160 g des in Beispiel I.2 beschriebenen Additionsproduktes gibt man 32 g 2-Ethylhexansäure und verrührt die Mischung bei Raumtemperatur homogen.
Kenndaten: Aminzahl: 121
Viskosität/25 °C: 14,3 Pa.s

II a) Herstellung einer lösungsmittelhaltigen Prepreg-Reaktionsmischung

Beispiel 1

Die bei Raumtemperatur hergestellte Lösung aus
14 g Härtungsmittel gemäß I.1 und
20 g Methylethylketon wird mit
100 g Epoxidharz auf Basis von Bisphenol A und einem Epoxidwert von 0,53
abgemischt und die Härtungscharakteristik dieses Systems mittels Differential Scannig Calorimetry (DSC) bestimmt; Gerät: Firma Mettler TA 3000 mit Meßzelle DSC 30.

Dazu werden ca. 25 mg des Reaktionsgemisches in einem Aluminium-Tiegel eingewogen und unter Stickstoff-Spülung ausgehend von 20 °C mit 10 C°/min erhitzt. Gemessen wird neben der Anspringtemperatur die Temperatur beim Auftreten der maximalen Wärmeabgabe (Peak-Temperatur) und die bei der Reaktion freigesetzte Wärmemenge. Die Anspringtemperatur dient unter anderem als Maß für die beim Abdampfen des Lösungsmittels maximal zulässige Temperaturbelastung (ohne daß die Härtungsreaktion nennenswert einsetzt) und sollte im Hinblick auf eine möglichst vollständige Lösungsmittelentfernung nicht zu gering liegen. Die Peak-Temperatur bestimmt das für die Materialaushärtung notwendige Temperaturniveau, so daß dieser Parameter aus verarbeitungstechnischen Gründen möglichst nicht zu hoch und dicht bei der Anspringtemperatur liegen sollte. Die bei der Reaktion freigesetzte Exothermie sollte zur Vermeidung einer übermäßigen thermischen Belastung des Materials bei der Aushärtung bzw. von großen Temperaturgradienten ebenfalls nicht zu hoch sein.

Die Meßdaten von Beispiel 1 sind in Tabelle 1 aufgeführt. Die ebenfalls in Tabelle 1 enthaltenen Daten der anderen Beispiele sind analog Beispiel 1 ermittelt.

Bei dem Vergleichsbeispiel handelt es sich um eine standardmäßige Prepreg-Reaktionsharzmischung mit folgender Härterzusammensetzung:
8 g Dicyandiamid (als latenter Härter)
2 g N-Methylimidazol (als Beschleuniger)

Tabelle 1

| Reaktionsspezifische Daten verchiedener lösungsmittelhaltiger Prepreg-Reaktionsmischungen nach DSC | | | | | |
|---|---|---|---|---|---|
| Beispiel | 1 | 2 | 3 | 4 | Vergleichsbeispiel |
| Härter | I.1 | I.2 | I.3 | I.4 | Dicyandiamid N-Methylimidazol |
| Anspringtemperatur °C | 105 | 110 | 95 | 115 | 100 |
| Peak-Temperatur °C | 141 | 140 | 135 | 148 | 148 |
| Exothermie ΔH J/g | 288 | 260 | 370 | 152 | 354 |

II b) Herstellung einer lösungsmittelfreien Prepreg-Reaktionsmischung

Beispiel 1

100 g eines Epoxidharzes (Epoxid-Äquivalentgewicht ca. 190) werden mit 7 g des erfindungsgemäßen Reaktionsprodukts I.1 vermischt und zur Prepreg-Herstellung eingesetzt. Diese Mischung besitzt bei Raumtemperatur eine Viskosität von 11,0 Pa.s und ist auch nach 10 h noch verarbeitungsfähig.

Die Herstellung der Prepregs im Labormaßstab erfolgt durch Aufstreichen des Reaktionsgemisches auf ein ca. 0,1 $m^2$ großes Glasfilamentgewebe in Atlasbindung, welches nach der Imprägnierung beidseitig mit Trennfolien kaschiert und bei Raumtemperatur gelagert wird.

Nach einer Zwischenlagerung von 24 h bei Raumtemperatur ist das Material soweit gereift, daß es als schwach klebriger Prepreg in mehreren Lagen im Heißpreßverfahren bei 0,1 bar und Temperaturen von 100 - 120 °C in 30 min bis 1 h zu hochfesten Formteilen weiterverarbeitet werden kann. Das derart ausgehärtete Endprodukt zeigt keinerlei Mängel bezüglich der Haftung der einzelnen Prepreglagen.

Die Ermittlung der in Tabelle 2 angegebenen Lagerstabilität erfolgt in Anlehnung an praxisgerechte Bedingungen. Das imprägnierte Gewebe wird zwischen zwei Polyethylenfolien bei 23 °C unter Normklima gelagert. In Abständen von 24 h wird eine Lage einer Probe unter praxisgerechten Bedingun gen (1 h, 120 °C, 0,1 bar) verpreßt. Als Lagerstabilität wird der Tag bestimmt, an dem das Harz letztmalig unter Preßbedingungen fließfähig ist. Analog Beispiel 1 sind die weiteren in der Tabelle 2 angeführten Lagerstabilitäten ermittelt.

Tabelle 2

| Lagerstabilitäten lösungsmittelfreier Prepregs | | | | |
|---|---|---|---|---|
| Beispiel | Härtungsmittel Beispiel | g Härtungsmittel | 100 g Epoxidharz | Lagerstabilität (Tage) |
| 1 | I.1 | 7 | Bisphenol A, Ep-Wert 0,53 | > 21 |
| 2 | I.1 | 14 | Bisphenol A, Ep-Wert 0,53 | 8 |
| 3 | I.1 | 28 | Bisphenol A, Ep-Wert 0,53 | 6 |
| 4 | I.2 | 7 | Bisphenol A, Ep-Wert 0,53 | > 21 |
| 5 | I.2 | 14 | Bisphenol A, Ep-Wert 0,53 | > 35 |
| 6 | 1.2 | 28 | Bisphenol A, Ep-Wert 0,53 | > 21 |
| 7 | 1.3 | 14 | Bisphenol A, Ep-Wert 0,53 | > 28 |
| 8 | 1.4 | 14 | Bisphenol A, Ep-Wert 0,53 | > 28 |

III. Ermittlung der Härtungsmitteleinflüsse

Zur Ermittlung der Härtungsmitteleigenschaften in Abhängigkeit der jeweiligen Struktur werden zur Vermeidung verfälschender Einflüsse von Verstärkungs- und Zusatzmaterialien die Mischungen, bestehend allein aus Epoxidharz und Härtungsmittel, ausgehärtet und abgeprüft.

In den in der Tabelle 3 aufgelisteten Beispielen wird als Epoxidharz ein Glycidylether auf Basis von Bisphenol A mit einem Epoxidwert von 0,53 verwendet.

Zur Herstellung der Prüfkörper werden jeweils 100 g des Epoxidharzes mit den in der Tabelle 3 genannten Mengen von Härtungsmitteln bei Raumtemperatur vermischt und in einem Stahlwerkzeug unter den angegebenen Bedingungen zu 4 mm dicken ebenen Formteilen ausgehärtet. Aus diesen Formteilen werden dann durch Sägen bzw. Fräsen Probekörper entnommen, an denen unter Einhaltung der nachstehend genannten Prüfnormen die in Tabelle 3 aufgeführten Eigenschaftswerte ermittelt sind.

| | Prüfkörperabmessungen: |
|---|---|
| Biegefestigkeit DIN 53 452 | 80 x 10 x 4 $mm^3$ |
| Durchbiegung DIN 53 452 | 80 x 10 x 4 $mm^3$ |
| Schlagzähigkeit DIN 53 453 | 50 x 6 x 4 $mm^3$ |
| Zugfestigkeit DIN 53 455 | Schulterstab Nr. 3 |
| Dehnung DIN 53 455 | Schulterstab Nr. 3 |
| Elastizitätsmodul DIN 53 457 | Schulterstab Nr. 3 |
| Heat Distortion Temperature (HDT) DIN 53 461 | Prüfkörperabmessungen 120 x 10 x 4 $mm^3$ |

Tabelle 3 Thermische und mechanische Eigenschaften

| Beispiel | 1 | 2 | 3 | 4 |
|---|---|---|---|---|
| Härtungsmittel | I.1 | I.1 | I.1 | I.1 |
| g Härtungsmittel | 7 | 14 | 14 | 14 |
| Härtungsbedingungen | 2h 120°C | 2h 80°C | 2h 100°C | 2h 120°C |
| Biegefestigkeit | 65 | 68 | 58 | 49 |
| Durchbiegung | 4,8 | 5,9 | 5,6 | 5,4 |
| Schlägzähigkeit | 6 | 4 | 3 | 3 |
| Zugfestigkeit | 22 | 39 | 29 | 30 |
| Dehnung | 0,7 | 1,5 | 1,2 | 1,3 |
| Elastizitätsmodul | 3250 | 3040 | 2460 | 2330 |
| HDT | 89 | 77 | 114 | 122 |

| Beispiel | 5 | 6 | 7 | 8 |
|---|---|---|---|---|
| Härtungsmittel | I.2 | I.2 | I.2 | I.2 |
| g Härtungsmittel | 7 | 10 | 14 | 14 |
| Härtungsbedingungen | 2h 120°C | 2h 120°C | 2h 80°C | 2h 100°C |
| Biegefestigkeit | 119 | 89 | 93 | 85 |
| Durchbiegung | 9,5 | 6,7 | 11,5 | 10,2 |
| Schlägzähigkeit | 14 | 17 | 16 | 13 |
| Zugfestigkeit | 74 | 29 | 41 | 29 |
| Dehnung | 3,4 | 1,5 | 1,6 | 1,3 |
| Elastizitätsmodul | 3030 | 1680 | 2730 | 2410 |
| HDT | 70 | 109 | 88 | 107 |

<u>Tabelle 3</u> (Fortsetzung)

| Beispiel | 9 | 10 | 11 | 12 |
|---|---|---|---|---|
| Härtungsmittel | I.2 | I.3 | I.4 | I.5 |
| g Härtungsmittel | 14 | 14 | 14 | 14 |
| Härtungsbedingungen | 2h 120°C | 2h 120°C | 2h 120°C | 2h 120°C |
| Biegefestigkeit | 59 | 91 | 85 | 86 |
| Durchbiegung | 6,0 | 7,9 | 12,9 | 14 |
| Schlägzähigkeit | 4 | 14 | 13 | 17 |
| Zugfestigkeit | 28 | 64 | 26 | 57 |
| Dehnung | 1,2 | 3,8 | 1,1 | 4,0 |
| Elastizitätsmodul | 2360 | 2430 | 2430 | 2290 |
| HDT | 120 | 122 | 101 | 108 |

**Ansprüche**

1. Verbindungen der allgemeinen Formel (I)

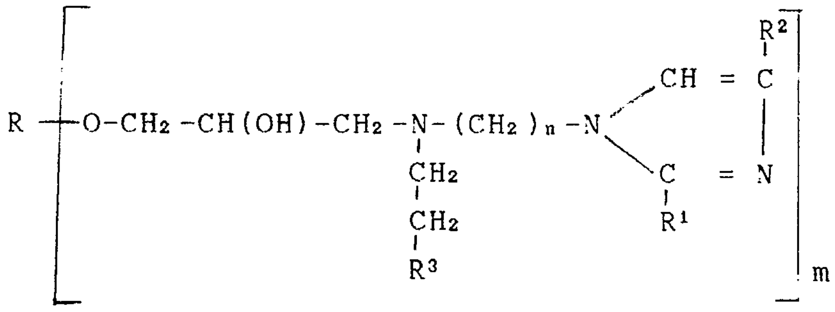

(I)

worin R der Rest der zur Herstellung der Glycidylether verwendeten Alkoholkomponente ist, $R^1$ und $R^2$ unabhängig voneinander Wasserstoff, $-CH_3$, $C_2H_5$ bedeuten und $_n$ = 2 oder 3 ist, $R^3$ = COOH, -CN, $-CONH-NH_2$, $-COOCH_2-CH_2-OH$, $-COOR^4$ mit $R^4$ = aliphatischer Kohlenwasserstoffrest mit 1 - 4 C-Atomen und m gleich der Wertigkeit von R ist.

2. Verwendung von Verbindungen der allgemeinen Formel (I)

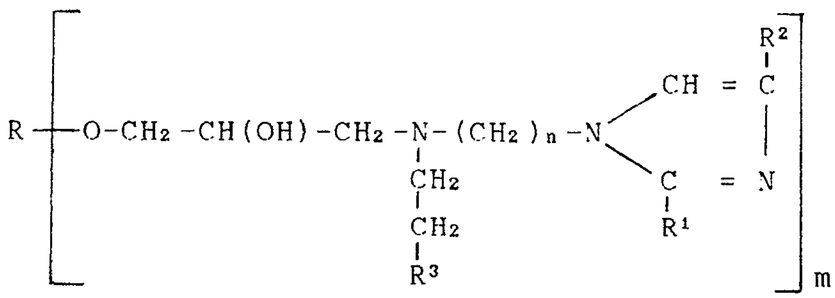

(I)

worin R der Rest der zur Herstellung der Glycidylether verwendeten Alkoholkomponente ist, $R^1$ und $R^2$ unabhängig voneinander Wasserstoff, $-CH_3$, $C_2H_5$ bedeuten und $_n$ = 2 oder 3 ist, $R^3$ = COOH, -CN, $-CONH-NH_2$, $-COOCH_2-CH_2-OH$, $-COOR^4$ mit $R^4$ = aliphatischer Kohlenwasserstoffrest mit 1 - 4 C-Atomen und m gleich der Wertigkeit von R ist, gegebenenfalls unter Mitverwendung üblicher stickstoffhaltiger heterocyclischer Aminverbindungen als Härtungsmittel für Epoxidharze.

3. Härtbare Epoxidharz-Zusammensetzungen enthaltend

a) ein Epoxidharz mit durchschnittlich mehr als einer Epoxidgruppe pro Molekül und

b) Verbindungen der allgemeinen Formel (I)

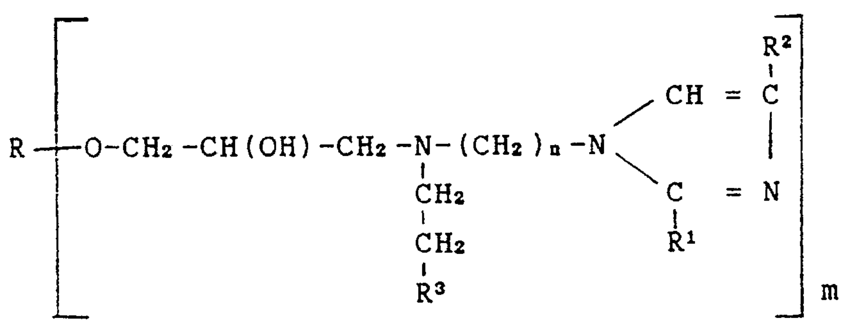

(I)

worin R der Rest der zur Herstellung der Glycidylether verwendeten Alkoholkomponente ist, $R^1$ und $R^2$ unabhängig voneinander Wasserstoff, $-CH_3$, $C_2H_5$ bedeuten und $_n$ = 2 oder 3 ist, $R^3$ = COOH, -CN, $-CONH-NH_2$, $-COOCH_2-CH_2-OH$, $-COOR^4$ mit $R^4$ = aliphatischer Kohlenwasserstoffrest mit 1 - 4 C-Atomen und m gleich der Wertigkeit von R ist und gegebenenfalls

c) übliche Lösungsmittel, Füllstoffe, Verstärkungs- oder Einlagematerialien, Pigmente, Hilfsstoffe und gegebenenfalls

d) übliche stickstoffhaltige heterocyclische Aminverbindungen.

4. Härtbare Epoxidharz-Zusammensetzungen, worin die Verstärkungs- oder Einlagematerialien bei Raumtemperatur mit dem Bindemittel, bestehend aus

a) einem Epoxidharz mit durchschnittlich mehr als einer Epoxidgruppe pro Molekül und

b) Verbindungen der allgemeinen Formel (I)

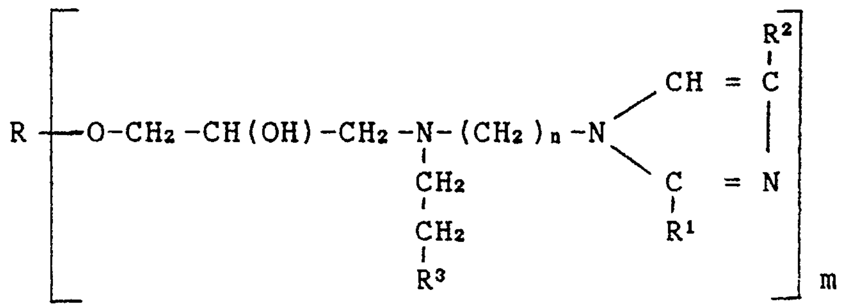

(I)

worin R der Rest der zur Herstellung der Glycidylether verwendeten Alkoholkomponente ist, $R^1$ und $R^2$ unabhängig voneinander Wasserstoff, $-CH_3$, $C_2H_5$ bedeuten und $_n$ = 2 oder 3 ist, $R^3$ = COOH, -CN, $-CONH-NH_2$, $-COOCH_2-CH_2-OH$, $-COOR^4$ mit $R^4$ = aliphatischer Kohlenwasserstoffrest mit 1 - 4 C-Atomen und m gleich der Wertigkeit von R ist und gegebenenfalls

c) Lösungsmitteln, Füllstoffen, Verstärkungs- oder Einlagematerialien, Pigmenten, Hilfsstoffen und gegebenenfalls

d) üblichen stickstoffhaltigen heterocyclischen Aminverbindungen

getränkt und gegebenenfalls bei erhöhter Temperatur in den halbfesten aber noch schmelzbaren Zustand (B-Zustand) überführt werden.

5. Epoxidharzformkörper, dadurch gekennzeichnet, daß in erster Stufe die Verstärkungs- oder Einlagematerialien bei Raumtemperatur mit dem Bindemittel, bestehend aus

a) einem Epoxidharz mit durchschnittlich mehr als einer Epoxidgruppe pro Molekül

b) Verbindungen der allgemeinen Formel (I)

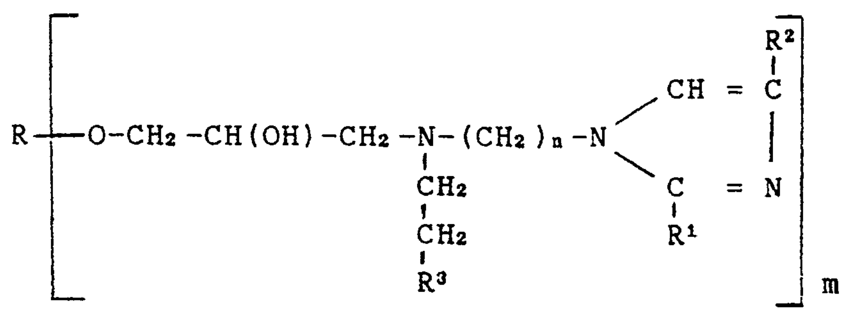

(I)

worin R der Rest der zur Herstellung der Glycidylether verwendeten Alkoholkomponente ist, $R^1$ und $R^2$ unabhängig voneinander Wasserstoff, $-CH_3$, $C_2H_5$ bedeuten und $_n$ = 2 oder 3 ist, $R^3$ = COOH, -CN, $-CONH-NH_2$, $-COOCH_2-CH_2-OH$, $-COOR^4$ mit $R^4$ = aliphatischer Kohlenwasserstoffrest mit 1 - 4 C-Atomen und m gleich der Wertigkeit von R ist und gegebenenfalls

c) übliche Lösungsmittel, Füllstoffe, Verstärkungs- und Einlagematerialien, Pigmente, Hilfsstoffe und gegebenenfalls

d) üblichen stickstoffhaltigen heterocyclischen Aminverbindungen

getränkt und gegebenenfalls in den halbfesten aber noch schmelzbaren Zustand (B-Zustand) überführt werden und in zweiter Stufe die feuchten Laminate oder Prepregs unter Formgebung oder zwischen zu verklebende Substrate gebracht, unter Anwendung von Druck bei erhöhter Temperatur ausgehärtet werden.

6. Verfahren zur Herstellung von faserverstärkten Trägermaterialien für den Elektrosektor durch Überführung von mit Bindemittel auf Basis von Epoxidharz und aminischen Härtungsmitteln imprägnierten Verstärkungsmaterialien in erster Stufe durch Anwendung von Druck und Temperatur in den B-Zustand und endgültiger Aushärtung bei erhöhter Temperatur, dadurch gekennzeichnet, daß als Härtungsmittel Verbindungen der allgemeinen Formel (I)

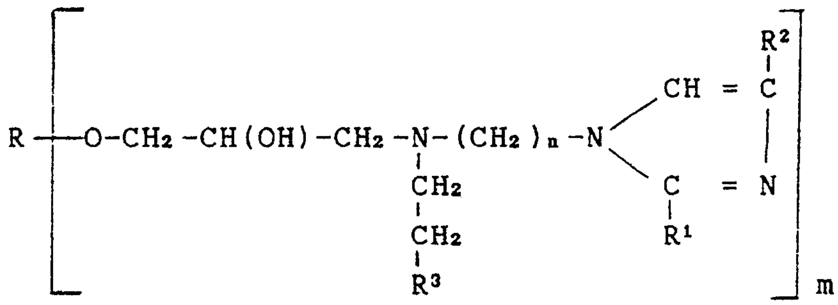 (I)

worin R der Rest der zur Herstellung der Glycidylether verwendeten Alkoholkomponente ist, $R^1$ und $R^2$ unabhängig voneinander Wasserstoff, $-CH_3$, $C_2H_5$ bedeuten und $_n$ = 2 oder 3 ist, $R^3$ = COOH, -CN, $-CONH-NH_2$, $-COOCH_2-CH_2-OH$, $-COOR^4$ mit $R^4$ = aliphatischer Kohlenwasserstoffrest mit 1 - 4 C-Atomen und m gleich der Wertigkeit von R ist, verwendet werden.

7. Verwendung von Verbindungen der allgemeinen Formel (I) als Härtungsmittel für Epoxidharze, dadurch gekennzeichnet, daß die Verbindungen der Formel (I) in Form ihrer Salze mit organischen und anorganischen Säuren eingesetzt werden.

8. Verwendung von Salzen gemäß Anspruch 7, dadurch gekennzeichnet, daß als Salzbildner verzweigte Monocarbonsäuren mit bis zu 10 C-Atomen verwendet werden.

Patentansprüche für folgenden Vertragsstaat: ES

1. Verfahren zur Herstellung von Verbindungen der allgemeinen Formel (I)

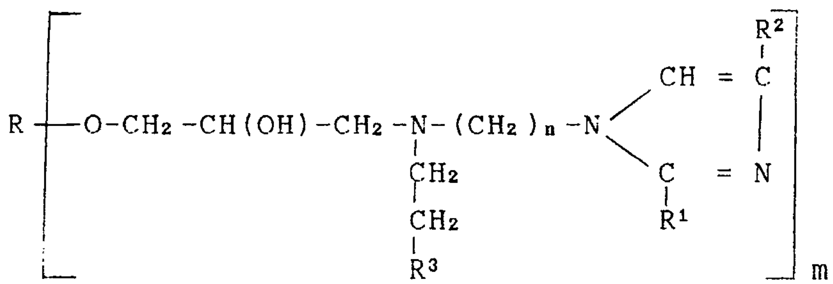 (I)

worin R der Rest der zur Herstellung der Glycidylether verwendeten Alkoholkomponente ist, $R^1$ und $R^2$ unabhängig voneinander Wasserstoff, $-CH_3$, $C_2H_5$ bedeuten und $_n$ = 2 oder 3 ist, $R^3$ = COOH, -CN, $-CONH-NH_2$, $-COOCH_2-CH_2-OH$, $-COOR^4$ mit $R^4$ = aliphatischer Kohlenwasserstoffrest mit 1 - 4 C-Atomen und m gleich der Wertigkeit von R ist, durch Additionsreaktion nach an sich bekannten Verfahren, wobei man in erster Stufe einen Ethergruppen enthaltenden Glycidylether mit primären Aminogruppen enthaltenden N-Aminoalkylimidazolyl-Verbindungen umsetzt, wobei das Verhältnis von Epoxidgruppen zu primären Aminogruppen ca. 1 : 1 beträgt, und in zweiter Stufe an die entstandenen sekundären Aminogruppen Acrylsäure oder deren Derivate addiert.

2. Verwendung von Verbindungen der allgemeinen Formel (I)

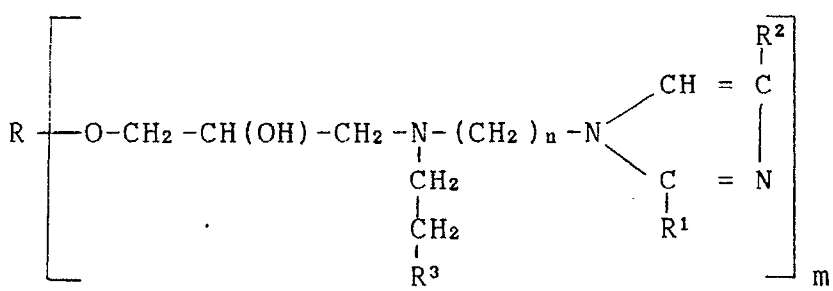 (I)

worin R der Rest der zur Herstellung der Glycidylether verwendeten Alkoholkomponente ist, $R^1$ und $R^2$

unabhängig voneinander Wasserstoff, $-CH_3$, $C_2H_5$ bedeuten und $_n$ = 2 oder 3 ist, $R^3$ = COOH, -CN, $-CONH-NH_2$, $-COOCH_2-CH_2-OH$, $-COOR^4$ mit $R^4$ = aliphatischer Kohlenwasserstoffrest mit 1 - 4 C-Atomen und m gleich der Wertigkeit von R ist, gegebenenfalls unter Mitverwendung üblicher stickstoffhaltiger heterocyclischer Aminverbindungen als Härtungsmittel für Epoxidharze.

3. Verfahren zur Herstellung härtbarer Epoxidharz-Zusammensetzungen durch Homogenisierung von härtbaren Epoxidharz-Zusammensetzungen enthaltend

a) ein Epoxidharz mit durchschnittlich mehr als einer Epoxidgruppe pro Molekül und

b) Verbindungen der allgemeinen Formel (I)

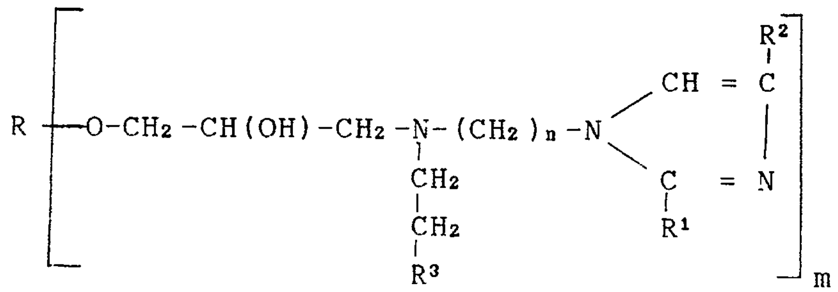

(I)

worin R der Rest der zur Herstellung der Glycidylether verwendeten Alkoholkomponente ist, $R^1$ und $R^2$ unabhängig voneinander Wasserstoff, $-CH_3$, $C_2H_5$ bedeuten und $_n$ = 2 oder 3 ist, $R^3$ = COOH, -CN, $-CONH-NH_2$, $-COOCH_2-CH_2-OH$, $-COOR^4$ mit $R^4$ = aliphatischer Kohlenwasserstoffrest mit 1 - 4 C-Atomen und m gleich der Wertigkeit von R ist und gegebenenfalls

c) übliche Lösungsmittel, Füllstoffe, Verstärkungs- oder Einlagematerialien, Pigmente, Hilfsstoffe und gegebenenfalls

d) übliche stickstoffhaltige heterocyclische Aminverbindungen.

4. Verfahren zur Herstellung härtbarer Epoxidharz-Zusammensetzungen, worin die Verstärkungs- oder Einlagematerialien bei Raumtemperatur mit dem Bindemittel, bestehend aus

a) einem Epoxidharz mit durchschnittlich mehr als einer Epoxidgruppe pro Molekül und

b) Verbindungen der allgemeinen Formel (I)

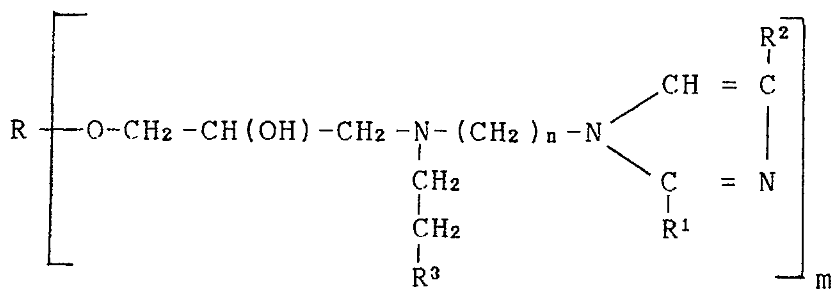

(I)

worin R der Rest der zur Herstellung der Glycidylether verwendeten Alkoholkomponente ist, $R^1$ und $R^2$ unabhängig voneinander Wasserstoff, $-CH_3$, $C_2H_5$ bedeuten und $_n$ = 2 oder 3 ist, $R^3$ = COOH, -CN, $-CONH-NH_2$, $-COOCH_2-CH_2-OH$, $-COOR^4$ mit $R^4$ = aliphatischer Kchlenwasserstoffrest mit 1 - 4 C-Atomen und m gleich der Wertigkeit von R ist und gegebenenfalls

c) Lösungsmitteln, Füllstoffen, Verstärkungs- oder Einlagematerialien, Pigmenten, Hilfsstoffen und gegebenenfalls

d) üblichen stickstoffhaltigen heterocyclischen Aminverbindungen

getränkt und gegebenenfalls bei erhöhter Temperatur in den halbfesten aber noch schmelzbaren Zustand (B-Zustand) überführt werden.

5. Verfahren zur Herstellung von Epoxidharzformkörpern, dadurch gekennzeichnet, daß in erster Stufe die Verstärkungs- oder Einlagematerialien bei Raumtemperatur mit dem Bindemittel, bestehend aus

a) einem Epoxidharz mit durchschnittlich mehr als einer Epoxidgruppe pro Molekül

b) Verbindungen der allgemeinen Formel (I)

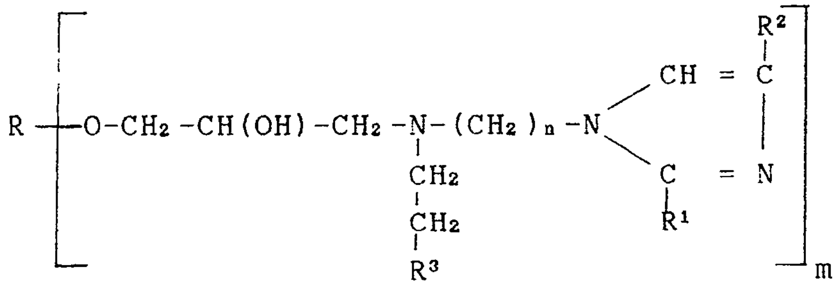

(I)

worin R der Rest der zur Herstellung der Glycidylether verwendeten Alkoholkomponente ist, $R^1$ und $R^2$ unabhängig voneinander Wasserstoff, $-CH_3$, $C_2H_5$ bedeuten und $_n$ = 2 oder 3 ist, $R^3$ = COOH, -CN, $-CONH-NH_2$, $-COOCH_2-CH_2-OH$, $-COOR^4$ mit $R^4$ = aliphatischer Kohlenwasserstoffrest mit 1 - 4 C-Atomen und m gleich der Wertigkeit von R ist und gegebenenfalls

c) übliche Lösungsmittel, Füllstoffe, Verstärkungs- und Einlagematerialien, Pigmente, Hilfsstoffe und gegebenenfalls

d) üblichen stickstoffhaltigen heterocyclischen Aminverbindungen

getränkt und gegebenenfalls in den halbfesten aber noch schmelzbaren Zustand (B-Zustand) überführt werden und in zweiter Stufe die feuchten Laminate oder Prepregs unter Formgebung oder zwischen zu verklebende Substrate gebracht, unter Anwendung von Druck bei erhöhter Temperatur ausgehärtet werden.

6. Verfahren zur Herstellung von faserverstärken Trägermaterialien für den Elektrosektor durch Überführung von mit Bindemittel auf Basis von Epoxidharz und aminischen Härtungsmitteln imprägnierten Verstärkungsmaterialien in erster Stufe durch Anwendung von Druck und Temperatur in den B-Zustand und endgültiger Aushärtung bei erhöhter Temperatur, dadurch gekennzeichnet, daß als Härtungsmittel Verbindungen der allgemeinen Formel (I)

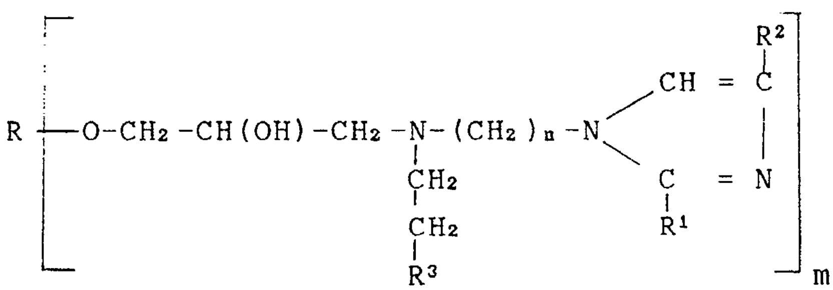

(I)

worin R der Rest der zur Herstellung der Glycidylether verwendeten Alkoholkomponente ist, $R^1$ und $R^2$ unabhängig voneinander Wasserstoff, $-CH_3$, $C_2H_5$ bedeuten und $_n$ = 2 oder 3 ist, $R^3$ = COOH, -CN, $-CONH-NH_2$, $-COOCH_2-CH_2-OH$, $-COOR^4$ mit $R^4$ = aliphatischer Kohlenwasserstoffrest mit 1 - 4 C-Atomen und m gleich der Wertigkeit von R ist, verwendet werden.

7. Verwendung von Verbindungen der allgemeinen Formel (I) als Härtungsmittel für Epoxidharze, dadurch gekennzeichnet, daß die Verbindungen der Formel (I) in Form ihrer Salze mit organischen und anorganischen Säuren eingesetz werden.

8. Verwendung von Salzen gemäß Anspruch 7, dadurch gekennzeichnet, daß als Salzbildner verzweigte Monocarbonsäuren mit bis zu 10 C-Atomen verwendet werden.